# EUROPEAN PATENT SPECIFICATION

(11) **EP 3 434 180 B1**
(45) Date of publication and mention of the grant of the patent: **08.01.2020**
(21) Application number: 18192797.1
(22) Date of filing: 08.06.2017
(51) Int. Cl.: A61B 5/00, A61B 5/042, A61B 5/06

(54) **DUAL-FUNCTION SENSORS FOR A BASKET CATHETER**
DOPPELFUNKTIONSSENSOREN FÜR EINEN KORBKATHETER
CAPTEURS À DOUBLE FONCTION POUR UN CATHÉTER À PANIER

(30) Priority: 09.06.2016 US 201615177775
(43) Date of publication of application: 30.01.2019
(62) Divisional of application: 17174936.9
(73) Proprietor: Biosense Webster (Israel) Ltd., Yokneam 2066717 (IL)
(72) Inventor: LEVIN, Michael, 2066717 Yokneam (IL); REUVENI, Avi, 2066717 Yokneam (IL); BAR-TAL, Meir, 2066717 Yokneam (IL); HIGHSMITH, Debby Esther, Irwindale, CA California 91706 (US); GARCIA, Ariel, Irwindale, CA California 91706 (US); OSADCHY, Daniel, 2066717 Yokneam (IL); AUERBACH, Shmuel, 2066717 Yokneam (IL)
(74) Representative: Small, Gary James

(56) References cited:
- EP-A1- 2 682 157
- WO-A1-2012/092016
- US-A- 5 411 025
- US-A1- 2011 264 000
- US-A1- 2015 057 519
- US-A1- 2015 119 670
- US-A1- 2015 250 424

## Description

### FIELD OF THE INVENTION

Embodiments of the present invention relate generally to the field of medical devices, and particularly to catheters for recording intracardiac electrocardiogram (ECG) signals.

### BACKGROUND

In some applications, a basket catheter, comprising a large number of electrodes disposed on a plurality of splines, is used to acquire intracardiac electrocardiogram (ECG) signals. Such signals may be used, for example, to construct an electroanatomical map of the heart.

US Patent Application Publication 2011/0118590, describes an interventional system for internal anatomical examination that includes a catheterization device for internal anatomical insertion. The catheterization device includes at least one magnetic field sensor for generating an electrical signal in response to rotational movement of the at least one sensor about an axis through the catheterization device within a magnetic field applied externally to patient anatomy, and a signal interface for buffering the electrical signal for further processing. A signal processor processes the buffered electrical signal to derive a signal indicative of angle of rotation of the catheterization device relative to a reference. The angle of rotation is about an axis through the catheterization device. A reproduction device presents a user with data indicating the angle of rotation of the catheterization device.

US Patent Application Publication 2003/0093067, describes systems and methods for imaging a body cavity and for guiding a treatment element within a body cavity. A system may include an imaging subsystem having an imaging device and an image processor that gather image data for the body cavity. A mapping subsystem may be provided, including a mapping device and a map processor, to identify target sites within the body cavity, and provide location data for the sites. The system may also include a location processor coupled to a location element on a treatment device to track the location of the location element. The location of a treatment element is determined by reference to the location element. A treatment subsystem including a treatment device having a treatment element and a treatment delivery source may also be provided. A registration subsystem receives and registers data from the other subsystems, and displays the data.

US Patent 6,272,371, describes an invasive probe apparatus including a flexible elongate probe having a distal portion adjacent to a distal end thereof for insertion into the body of a subject, which portion assumes a predetermined curve form when a force is applied thereto. First and second sensors are fixed to the distal portion of the probe in known positions relative to the distal end, which sensors generate signals responsive to bending of the probe. Signal processing circuitry receives the bend responsive signals and processes them to find position and orientation coordinates of at least the first sensor, and to determine the locations of a plurality of points along the length of the distal portion of the probe.

US Patent Application Publication 2006/0025677, describes a surgical navigation system for navigating a region of a patient that may include a non-invasive dynamic reference frame and/or fiducial marker, sensor tipped instruments, and isolator circuits. The dynamic reference frame may be placed on the patient in a precise location for guiding the instruments. The dynamic reference frames may be fixedly placed on the patient. Also the dynamic reference frames may be placed to allow generally natural movements of soft tissue relative to the dynamic reference frames. Also methods are provided to determine positions of the dynamic reference frames. Anatomical landmarks may be determined intra-operatively and without access to the anatomical structure.

US Patent 6,892,091, describes an apparatus and method for rapidly generating an electrical map of a chamber of a heart that utilizes a catheter including a body having a proximal end and a distal end. The distal end has a distal tip and an array of non-contact electrodes having a proximal end and a distal end and at least one location sensor. Preferably, two location sensors are utilized. The first location sensor is preferably proximate to the catheter distal tip and the second location sensor is preferably proximate to the proximal end of the non-contact electrode array. The catheter distal end further preferably includes a contact electrode at its distal tip. Preferably, at least one and preferably both of the location sensors provide six degrees of location information. The location sensor is preferably an electromagnetic location sensor. The catheter is used for rapidly generating an electrical map of the heart within at least one cardiac cycle and preferably includes cardiac ablation and post-ablation validation.

WO2012092016 (A1) relates to a system for diagnosing arrhythmias and directing catheter therapies may allow for measuring, classifying, analyzing, and mapping spatial electrophysiological (EP) patterns within a body.

US2011264000 (A1) relates to a method and system for determining tissue type is provided. The system comprises an electronic control unit (ECU) configured to acquire a value of an electrical parameter between a first electrode electrically coupled with tissue and a second electrode. The ECU is further configured to identify a tissue type from a plurality of tissue types based at least on the acquired value, and in an exemplary embodiment, to generate a tissue morphology map comprising a marker representative of the identified tissue type.

### SUMMARY OF THE INVENTION

The present invention is an apparatus according to claim 1. Further embodiments of the invention are described in the dependent claims.

The present invention will be more fully understood from the following detailed description of embodiments thereof, taken together with the drawings, in which:

### BRIEF DESCRIPTION OF THE DRAWINGS

Fig. 1 is a schematic illustration of a basket catheter, in accordance with some embodiments of the present invention; and
Figs. 2-3 are schematic illustrations of circuitry for processing signals received from conducting elements, in accordance with some embodiments of the present invention.

### DETAILED DESCRIPTION OF EMBODIMENTS

### OVERVIEW

Embodiments described herein include a basket catheter that may be used, for example, to build an electroanatomical map. The basket catheter comprises a plurality of splines at its distal end, and further comprises a plurality of helical conducting elements, which are disposed on the splines. During the electroanatomical mapping procedure, the helical conducting elements function as inductors, in that a generated magnetic field induces respective voltage differences across the conducting elements. Based on the induced voltage differences, the respective locations and orientations of the conducting elements - and hence, the location and orientation of the basket catheter - may be precisely determined.

Typically, embodiments described herein are rendered even more advantageous, in that the helical conducting elements may additionally function as electrodes for acquiring ECG signals, such that it may not be necessary to equip the basket catheter with separate ECG-acquiring electrodes. For example, an electrically-insulative layer may cover the majority of each of the helical conducting elements, but leave a small portion of each of the helical conducting elements exposed. This exposed portion, when brought into contact with the intracardiac tissue, acquires ECG signals from the tissue.

The helical conducting elements described herein may thus function in two capacities - e.g., simultaneously - during a single procedure. First, they may function as ECG electrodes, by sensing the intracardiac ECG signals. Second, they may function as magnetic-field sensors, by generating location signals (in the form of the above-described induced voltages) in response to the generated magnetic field. The conducting elements may thus be described as ECG electrodes that additionally function as magnetic-field sensors, or as magnetic-field sensors that additionally function as ECG electrodes. (Notwithstanding the above, in some embodiments, the conducting elements are used only as magnetic-field sensors, and separate electrodes coupled to the splines are used to acquire the ECG signals.)

Embodiments described herein further include circuitry for processing signals received from the helical conducting elements. In particular, the circuitry described herein generates, based on the received signals, a plurality of outputs, which are used by a processor to construct an electroanatomical map. These outputs include a plurality of first outputs, which indicate the electrical activity of the tissue, a plurality of second outputs, which indicate the respective induced voltage differences across the conducting elements, and a plurality of third outputs, which indicate the proximity to the tissue of each of the conducting elements.

### APPARATUS DESCRIPTION

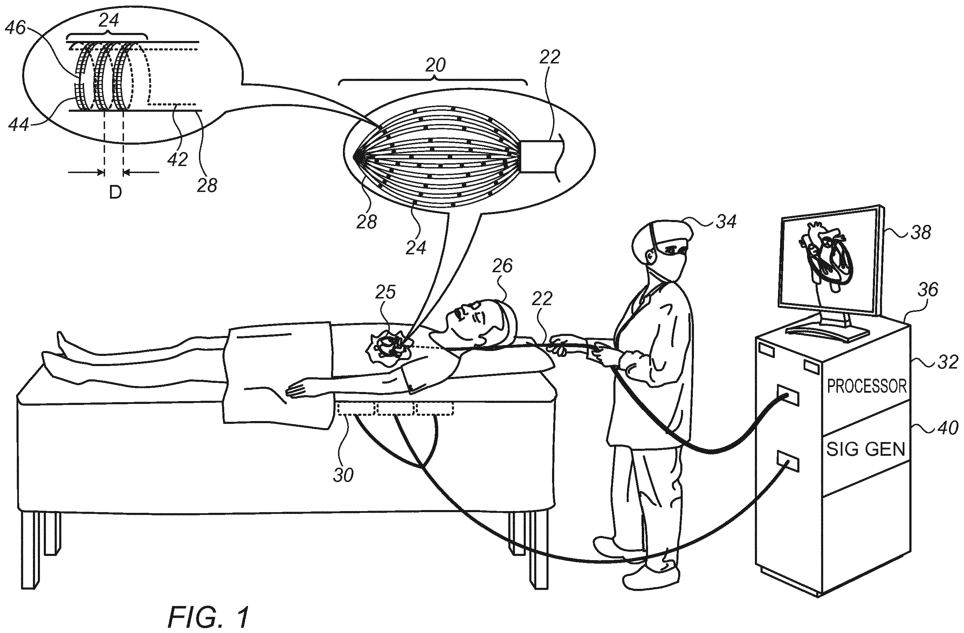

Reference is initially made to Fig. 1, which is a schematic illustration of a basket catheter 22, in accordance with some embodiments of the present invention. Fig. 1 depicts a physician 34 using basket catheter 22 to perform an electroanatomical mapping of a heart 25 of a subject 26. During the mapping procedure, the distal end of the catheter, which comprises a basket 20 of splines 28, is inserted into heart 25. The splines are then brought into contact with the intracardiac tissue, and conducting elements 24 on the splines acquire intracardiac ECG signals. A console 36, which is connected to the basket catheter and comprises a computer processor 32, receives these ECG signals.

While the intracardiac ECG signals are being acquired, a magnetic field is generated by a plurality of magnetic-field generators 30 located underneath subject 26 or otherwise in the vicinity of the subject. (As shown in Fig. 1, a signal generator ("SIG GEN") 40 in console 36 may cause generators 30 to generate the magnetic field by supplying an alternating current to the generators.) The magnetic field induces voltage differences across conducting elements 24. The induced voltage differences are received by the console, and, based on the induced voltages, processor 32 ascertains the position of each of the conducting elements. Processor 32 then constructs an electroanatomical map of the heart, based on the ECG signals (which indicate the electrical activity of the intracardiac tissue) and the voltages received from the helical conducting elements (which indicate the respective locations of the sources of the ECG signals). Such a map may be displayed on a monitor 38 for viewing by physician 34, and/or stored for later analysis.

Splines 28 may be arranged to define any suitably-shaped basket, such as the spheroidal basket shown in Fig. 1. Fig. 1 shows an embodiment in which a plurality of helical conducting elements 24 are disposed on the surface of each of the splines. The top-left portion of the figure shows an enlarged view of a single such helical conducting element. In this enlarged view, the solid portion of the conducting element corresponds to the portion of the conducting element that is on the near side of the spline, facing the viewer. The dotted portion corresponds to the portion of the conducting element that is on the far side of the spline, facing away from the viewer. Each of the two terminals of each of the conducting elements is typically connected to the console via a wire 42 which passes through the interior of the spline.

In some embodiments, the conducting elements are printed onto the splines. For example, each of the conducting elements may comprise electrically-conductive paint that is helically painted onto the splines. In other embodiments, the conducting elements comprise wires that are wound (i.e., coiled) around, and glued or otherwise attached to, the splines. In any case, for embodiments in which the helical conducting elements are on the surface of the splines, an electrically-insulative layer 44 typically covers at least a majority of each of the helical conducting elements. Electrically-insulative layer 44 prevents the turns of any given conducting element from being shorted with each other.

Typically, the electrically-insulative layer does not cover a portion of exactly one respective turn of each of the helical conducting elements. Thus, the electrically-insulative layer prevents shorting of the turns (in that no more than one turn of each conducting element is exposed), but also allows the conducting elements to acquire ECG signals. For example, the enlarged portion of Fig. 1 shows an embodiment in which the electrically-insulative layer exposes a portion 46 of the conducting element. Exposed portion 46 may be brought into contact with tissue, in order to acquire an ECG signal.

As noted above, the exposed portion of the conducting element is confined to one turn of the conducting element. This means that the distance between the distalmost exposed portion of the conducting element and the proximalmost exposed portion of the conducting element is less than the distance D that separates between successive turns of the conducting element.

In some embodiments, the electrically-insulative layer is contiguous across a plurality of conducting elements. In other embodiments, as depicted in Fig. 1, the electrically-insulative layer is discontiguous, such that no portion of the electrically-insulative layer covers more than one of the conducting elements. Similarly, for any given conducting element, the cover provided by the electrically-insulative layer may be contiguous or discontiguous. As an example of the latter, in Fig. 1, the conducting element is covered by two separate, disjoint portions of the electrically-insulative layer, these portion being on respective opposite sides of exposed portion 46 of the conducting element.

In some embodiments, alternatively to being disposed on the splines as in Fig. 1, the conducting elements are contained within the splines. In such embodiments, the splines, being made of an electrically-insulative material (such as plastic), provide the "cover" that prevents the conducting elements from being shorted. For embodiments in which the conducting elements are additionally used to acquire ECG signals, the splines are shaped to define a plurality of openings that expose a portion of exactly one respective turn of each of the helical conducting elements. In other words, such embodiments are analogous to the embodiments described above, with the surface of the spline functioning analogously to electrically-insulative layer 44 in preventing shorting of the conducting elements, but also, optionally, providing for ECG-signal acquisition.

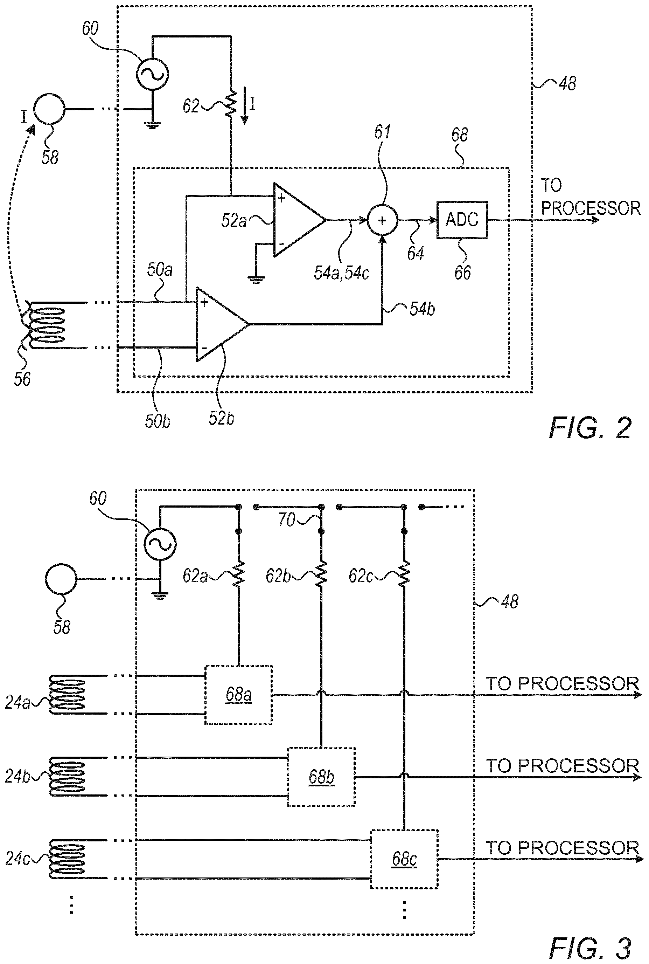

Reference is now made to Fig. 2, which is a schematic illustration of circuitry 48 for processing signals received from conducting elements 24, in accordance with some embodiments of the present invention. Circuitry 48 is typically located within console 36, between the catheter-console interface and the processor. As shown in Fig. 2, circuitry 48 is connected to each helical conducting element 24, typically via exactly two connections (or "leads") connected to the conducting element: a first connection 50a to one terminal of the conducting element, and a second connection 50b to the other terminal of the conducting element. As further described below, circuitry 48 generates outputs based on signals received, via connections 50a and 50b, from each helical conducting element. Based on these outputs, processor 32 constructs an electroanatomical map of the subject's heart.

Typically, circuitry 48 comprises a first differential amplifier 52a and a second differential amplifier 52b. Connections 50a and 50b are connected to second differential amplifier 52b, while one of the connections - e.g., first connection 50a - is also connected to first differential amplifier 52a. Connections 50a and 50b thus carry inputs to the differential amplifiers, as further described below.

As described above, the exposed portion of each conducting element 24 is brought into contact with intracardiac tissue 56, such that an ECG voltage (referred to above as an "ECG signal") is transferred to the conducting element from the tissue. (The ECG voltage is generally constant across the conducting element, i.e., the ECG voltage at the terminal of the conducting element is not significantly different from the ECG voltage at the exposed portion of the conducting element.) First connection 50a carries the ECG voltage to first differential amplifier 52a, which generates a first output 54a based on the ECG voltage, by amplifying a difference between the received ECG voltage and a reference voltage. The processor derives electrical-activity information from first output 54a, and uses this information to build the electroanatomical map. Typically, the reference voltage is the voltage at a reference electrode 58 disposed on the basket catheter, e.g., on a central spline of the catheter shaft (not shown in Fig. 1). (In Fig. 2, reference electrode 58 is connected to ground, such that the reference voltage is ground.)

Connection 50a also carries, to second differential amplifier 52b, the voltage induced by the magnetic field at one terminal of the conducting element, while connection 50b carries the voltage induced at the other terminal. In other words, connections 50a and 50b collectively carry, to the second differential amplifier, the voltage difference that is induced across the conducting element. Based on this voltage difference, second differential amplifier 52b generates a second output 54b, by amplifying the voltage difference. Second output 54b includes anatomical information, in that the second output indicates the position of the conducting element, and hence, the location of the source of the ECG signal. The processor derives this anatomical information from the second output, and then, in building the electroanatomical map, combines this anatomical information with the electrical-activity information derived from the first output.

Typically, circuitry 48 further comprises a current source, or, as in Fig. 2, a voltage source 60 in series with a resistor 62, which together function as a current source. The current source passes a current "I" over connection 50a and between the conducting element and reference electrode 58 (or a different reference electrode that is not used for the ECG reference voltage). During the passing of the current, the voltage on the conducting element indicates the impedance that is seen by the conducting element; the higher the voltage, the higher the impedance. The impedance, in turn, indicates the proximity of the conducting element to the tissue; the higher the impedance, the greater the proximity. Thus, the voltage on the conducting element indicates the proximity of the conducting element to the tissue. The first differential amplifier generates a third output 54c based on this proximity-indicating voltage, by amplifying the difference between the proximity-indicating voltage and the reference voltage. The processor then uses the third output to build the electroanatomical map. In particular, the processor first derives, from the third output, the proximity of the conducting element to the tissue. The processor then decides whether to accept the first (electrical-activity-related) output, based on the proximity. For example, the processor may compare the proximity to a threshold, and accept the first output only if the proximity is greater than the threshold (i.e., the distance between the conducting element and the tissue is sufficiently small).

It is noted that the ECG voltage, the induced voltage, and the proximity-indicating voltage are of sufficiently different frequencies, such that the three voltages may be simultaneously carried on connection 50a (and hence, simultaneously received by the circuitry). Thus, first output 54a, second output 54b, and third output 54c may be generated at the same time. In some embodiments, an adder 61 adds the first output, the second output, and the third output, yielding a combined output 64 having a plurality of components at various frequencies. Combined output 64 is then passed to an analog-to-digital converter (ADC) 66, which converts the combined output to a digital signal that is passed to the processor.

Although, for simplicity, only a single helical conducting element 24 is shown in Fig. 2, basket catheter 22 typically comprises a large number of helical conducting elements. On this note, reference is now made to Fig. 3, which is a schematic illustration of circuitry 48, in accordance with some embodiments of the present invention.

Fig. 3 shows a way in which the configuration of circuitry 48 shown in Fig. 2 may be extended to handle a large number of inputs from a large number of helical conducting elements. In particular, in Fig. 3, a block 68 of circuitry that is shown in Fig. 2 is replicated for each of the conducting elements. Thus, in Fig. 3, a conducting element 24a connects to a block 68a of circuitry, a conducting element 24b connects to a block 68b, and a conducting element 24c connects to a block 68c. Similarly, resistor 62 is replicated for each of the conducting elements, such that voltage source 60 may be connected to block 68a via a resistor 62a, to block 68b via a resistor 62b, or to block 68c via a resistor 62c. (Typically, switches 70 ensure that the voltage source is connected to no more than one block at a time.) Thus, for example, to pass a current between conducting element 24a and the reference electrode, the voltage source is connected to block 68a.

As indicated by the three-dot sequences in the figure, the configuration shown in Fig. 3 may be extended to handle any number of conducting elements.

It is emphasized that the principles described herein may be applied in many ways. For example, the scope of the present disclosure includes using each of one or more coils, and/or other conducting elements, for both (i) magnetic tracking, and (ii) exchanging signals with tissue, in any relevant application. (Circuitry described with reference to Figs. 2-3 may be modified as appropriate to suit the application.) Exchanging signals with tissue includes, for example, acquiring ECG signals as described above, and/or passing ablating signals into tissue. (In the latter case, the same leads that carry the induced voltage from the conducting element may be used to deliver the ablating signal to the conducting element.) Moreover, the dual-function sensors described herein may be disposed on any suitable apparatus, including, for example, a lasso catheter, balloon catheter, or other type of catheter.

It will be appreciated by persons skilled in the art that the present invention is not limited to what has been particularly shown and described hereinabove. Rather, the scope of embodiments of the present invention, the invention being an apparatus according to claim 1, includes both combinations and subcombinations of the various features described hereinabove, as well as variations and modifications thereof that are not in the prior art, which would occur to persons skilled in the art upon reading the foregoing description.

## Claims

1. Apparatus, comprising:
Circuitry (48), configured:
to generate a first output (54a), based on an intracardiac electrocardiogram (ECG) voltage received from a helical conducting element (24), and
to generate a second output (54b), based on a voltage difference that was induced across the conducting element by a magnetic field;
a processor (32), configured to build an electroanatomical map, based on the first output and the second output; and
a catheter (22), the catheter comprising:
a plurality of splines (28) at a distal end of the catheter; and
a plurality of helical conducting elements (24) disposed on the splines;
wherein the circuitry is configured to generate the first output based on an intracardiac electrocardiogram (ECG) voltage received from a first one of the plurality of helical conducting elements, and the circuitry is configured to generate the second output based on a voltage difference that was induced across the first one of the plurality of helical conducting elements by a magnetic field; and
wherein the apparatus further comprises an electrically-insulative layer (44) covering at least a majority of each of the helical conducting elements.

2. The apparatus according to claim 1, wherein the circuitry is further configured:
to cause a proximity-indicating voltage to be received from the conducting element, by passing a current between the conducting element and a reference electrode, and
to generate a third output (54c), based on the proximity-indicating voltage, and
wherein the processor is configured to build the electroanatomical map based on the third output.

3. The apparatus according to claim 2, wherein the processor is configured to derive, from the third output, a proximity of the conducting element to tissue.

4. The apparatus according to claim 2, wherein the circuitry comprises:
a first differential amplifier (52a), configured:
to generate the first output by amplifying a difference between the ECG voltage and a reference voltage, and
to generate the third output by amplifying a difference between the proximity-indicating voltage and the reference voltage; and
a second differential amplifier (52b), configured to generate the second output by amplifying the induced voltage difference.

5. The apparatus according to claim 1, wherein the circuitry comprises exactly two connections (50a, 50b) to the conducting element.

6. The apparatus according to claim 1, wherein the processor is configured:
to derive electrical-activity information from the first output,
to derive anatomical information from the second output, and
to build the electroanatomical map by combining the electrical-activity information with the anatomical information.

7. The apparatus according to claim 1, wherein the first output is one of a plurality of first outputs and the second output is one of a plurality of second outputs, wherein the circuitry is configured to generate the plurality of first outputs, each of the plurality of first outputs based on an intracardiac electrocardiogram (ECG) voltage received from a respective one of the plurality of helical conducting elements, wherein the circuitry is configured to generate the plurality of second outputs, each of the plurality of second outputs based on a voltage difference that was induced across a respective one of the plurality of helical conducting elements by a magnetic field, and wherein the processor is configured to build the electroanatomical map based on the plurality of first outputs and the plurality of second outputs.

8. The apparatus according to claim 1 or 7, wherein the plurality of splines are arranged to define a basket.

9. The apparatus according to claim 1 or 7, wherein the helical conducting elements are printed onto the splines.

10. The apparatus according to claim 9, wherein each of the helical conducting elements comprises electrically-conductive paint that is helically painted onto the splines.

11. The apparatus according to claim 1, wherein the electrically-insulative layer does not cover a portion of exactly one respective turn of each of the helical conducting elements.

## Patentansprüche

1. Vorrichtung, umfassend:
Schaltung (en) (48), konfiguriert:
zum Generieren einer ersten Ausgabe (54a) basierend auf der Spannung eines intrakardialen Elektrokardiogramm (EKG), die von dem wendelförmigen leitfähigen Element (24) empfangen wird, und
zum Generieren einer zweiten Ausgabe (54b) basierend auf einer Spannungsdifferenz, die durch ein Magnetfeld über dem leitfähigen Element induziert wurde;
einen Prozessor (32), der konfiguriert ist zum Aufbauen eines elektroanatomischen Maps basierend auf der ersten Ausgabe und der zweiten Ausgabe; und
einen Katheter (22), wobei der Katheter umfasst:
eine Vielzahl von Latten ("Splines") (28) an einem distalen Ende der Katheter; und
eine Vielzahl von wendelförmigen leitfähigen Elementen (24), die auf den Splines angeordnet sind;
wobei die Schaltung konfiguriert ist zum Generieren der ersten Ausgabe basierend auf einer Spannung des intrakardialen Elektrokardiogramms (EKG), die von einem ersten von der Vielzahl der wendelförmigen leitfähigen Elemente empfangen wird, und wobei die Schaltung konfiguriert ist zum Generieren der zweiten Ausgabe basierend auf einer Spannungsdifferenz, die durch ein Magnetfeld über dem ersten von der Vielzahl der wendelförmigen leitfähigen Elemente induziert wurde; und
wobei die Vorrichtung ferner eine elektrisch isolierende Schicht (44) umfasst, die mindestens den überwiegenden Teil von jedem der wendelförmigen leitfähigen Elemente bedeckt.

2. Vorrichtung nach Anspruch 1, wobei die Schaltung ferner konfiguriert ist:
zum Herbeiführen, dass eine Nähe anzeigende Spannung von dem leitfähigen Element empfangen wird, indem ein Strom zwischen dem leitfähigen Element und einer Referenzelektrode geleitet wird, und
zum Generieren einer dritten Ausgabe (54c) basierend auf der Nähe anzeigenden Spannung, und wobei der Prozessor konfiguriert ist zum Aufbauen des elektroanatomischen Maps basierend auf der dritten Ausgabe.

3. Vorrichtung nach Anspruch 2, wobei der Prozessor konfiguriert ist zum Ableiten einer Nähe des leitfähigen Elements zu Gewebe aus der dritten Ausgabe.

4. Vorrichtung nach Anspruch 2, wobei die Schaltung umfasst:
einen ersten Differentialverstärker (52a), der konfiguriert ist:
zum Generieren der ersten Ausgabe durch Verstärken einer Differenz zwischen der EKG-Spannung und einer Referenzspannung, und
zum Generieren der dritten Ausgabe durch Verstärken einer Differenz zwischen der Nähe anzeigenden Spannung und der Referenzspannung; und
einen zweiten Differentialverstärker (52b), der konfiguriert ist zum Generieren der zweiten Ausgabe, indem die induzierte Spannungsdifferenz verstärkt wird.

5. Vorrichtung nach Anspruch 1, wobei die Schaltung(en) genau zwei Verbindungen (50a, 50b) mit dem leitfähigen Element umfasst bzw. umfassen.

6. Vorrichtung nach Anspruch 1, wobei der Prozessor konfiguriert ist:
zum Ableiten von elektrischen Aktivitätsinformationen aus der ersten Ausgabe, zum Ableiten von anatomischen Informationen aus der zweiten Ausgabe, und
zum Aufbauen des elektroanatomischen Maps, indem die elektrischen Aktivitätsinformationen mit den anatomischen Informationen kombiniert werden.

7. Vorrichtung nach Anspruch 1, wobei die erste Ausgabe eine von einer Vielzahl von ersten Ausgaben ist, und die zweite Ausgabe eine von einer Vielzahl von zweiten Ausgaben ist, wobei die Schaltung(en) konfiguriert ist bzw. sind zum Generieren der Vielzahl von ersten Ausgaben, wobei jede von der Vielzahl der ersten Ausgaben auf der Spannung in einem intrakardialen Elektrokardiogramm (EKG) basiert, die von jeweils einem von der Vielzahl der wendelförmigen leitfähigen Elemente empfangen wird, wobei die Schaltung(en) konfiguriert ist bzw. sind zum Generieren der Vielzahl von zweiten Ausgaben, wobei jede von der Vielzahl der zweiten Ausgaben auf einer Spannungsdifferenz basiert, die durch ein Magnetfeld über jeweils einem der wendelförmigen leitfähigen Elemente induziert wurde, und wobei der Prozessor konfiguriert ist, um das elektroanatomische Map basierend auf der Vielzahl der ersten Ausgaben und der Vielzahl der zweiten Ausgaben aufzubauen.

8. Vorrichtung nach Anspruch 1 oder 7, wobei die Vielzahl der Splines so angeordnet ist, dass sie einen Korb definieren.

9. Vorrichtung nach Anspruch 1 oder 7, wobei die wendelförmigen leitfähigen Elemente auf die Splines gedruckt sind.

10. Vorrichtung nach Anspruch 9, wobei jedes der wendelförmigen leitfähigen Elemente elektrisch leitfähige Farbe umfasst, die wendelförmig auf die Splines gemalt wird.

11. Vorrichtung nach Anspruch 1, wobei die elektrisch isolierende Schicht nicht einen Abschnitt von genau einer jeweiligen Windung von jedem der wendelförmigen leitfähigen Elemente bedeckt.

## Revendications

1. Appareil, comprenant :
un circuit (48), configuré :
pour générer une première sortie (54a), sur la base d'une tension d'électrocardiogramme intracardiaque (ECG) reçue d'un élément conducteur hélicoïdal (24), et
pour générer une deuxième sortie (54b), sur la base d'une différence de tension qui a été induite à travers l'élément conducteur par un champ magnétique ;
un processeur (32), configuré pour construire une carte électro-anatomique, sur la base de la première sortie et de la deuxième sortie ; et
un cathéter (22), le cathéter comprenant :
une pluralité de cannelures (28) au niveau d'une extrémité distale du cathéter ; et
une pluralité d'éléments conducteurs hélicoïdaux (24) disposés sur les cannelures ;
le circuit étant configuré pour générer la première sortie sur la base d'une tension d'électrocardiogramme intracardiaque (ECG) reçue d'un premier élément de la pluralité d'éléments conducteurs hélicoïdaux, et le circuit étant configuré pour générer la deuxième sortie sur la base d'une différence de tension qui a été induite à travers le premier élément de la pluralité d'éléments conducteurs hélicoïdaux par un champ magnétique ; et
l'appareil comprenant en outre une couche électriquement isolante (44) recouvrant au moins une majorité de chacun des éléments conducteurs hélicoïdaux.

2. Appareil selon la revendication 1, le circuit étant en outre configuré :
pour amener une tension d'indication de proximité à être reçue de l'élément conducteur, en faisant passer un courant entre l'élément conducteur et une électrode de référence, et
pour générer une troisième sortie (54c), sur la base de la tension d'indication de proximité, et
le processeur étant configuré pour construire la carte électro-anatomique sur la base de la troisième sortie.

3. Appareil selon la revendication 2, le processeur étant configuré pour dériver, à partir de la troisième sortie, une proximité de l'élément conducteur au tissu.

4. Appareil selon la revendication 2, les circuits comprenant :
un premier amplificateur différentiel (52a), configuré :
pour générer la première sortie en amplifiant une différence entre la tension ECG et une tension de référence, et
pour générer la troisième sortie en amplifiant une différence entre la tension d'indication de proximité et la tension de référence ; et
un deuxième amplificateur différentiel (52b), configuré pour générer la deuxième sortie en amplifiant la différence de tension induite.

5. Appareil selon la revendication 1, le circuit comprenant exactement deux connexions (50a, 50b) à l'élément conducteur.

6. Appareil selon la revendication 1, le processeur étant configuré :
pour dériver des informations sur l'activité électrique à partir de la première sortie,
pour dériver des informations anatomiques à partir de la deuxième sortie, et
pour construire la carte électro-anatomique en combinant les informations sur l'activité électrique avec les informations anatomiques.

7. Appareil selon la revendication 1, la première sortie étant l'une d'une pluralité de premières sorties et la deuxième sortie étant l'une d'une pluralité de deuxièmes sorties, le circuit étant configuré pour générer la pluralité de premières sorties, chacune de la pluralité de premières sorties sur la base d'une tension d'électrocardiogramme intracardiaque (ECG) reçue d'un élément conducteur respectif parmi la pluralité d'éléments conducteurs hélicoïdaux, le circuit étant configuré pour générer la pluralité de deuxièmes sorties, chacune de la pluralité de deuxièmes sorties sur la base d'une différence de tension qui a été induite à travers un élément respectif de la pluralité d'éléments conducteurs hélicoïdaux par un champ magnétique, et le processeur étant configuré pour construire la carte électro-anatomique sur la base de la pluralité des premières sorties et de la pluralité des deuxièmes sorties.

8. Appareil selon la revendication 1 ou 7, la pluralité de cannelures étant agencées pour définir un panier.

9. Appareil selon la revendication 1 ou 7, les éléments conducteurs hélicoïdaux étant imprimés sur les cannelures.

10. Appareil selon la revendication 9, chacun des éléments conducteurs hélicoïdaux comprenant une peinture électriquement conductrice qui est peinte en hélice sur les cannelures.

11. Appareil selon la revendication 1, la couche électriquement isolante ne recouvrant pas une partie d'exactement un tour respectif de chacun des éléments conducteurs hélicoïdaux.
